# EUROPÄISCHE PATENTSCHRIFT

(11) **EP 0 672 076 B1**
(45) Veröffentlichungstag und Bekanntmachung des Hinweises auf die Patenterteilung: **22.10.1997**
(21) Anmeldenummer: 94901874.1
(22) Anmeldetag: 25.11.1993
(51) Int. Cl.: C08G 18/32, C08G 18/65, A61K 7/00

(54) **VERWENDUNG VON KATIONISCHEN POLYURETHANEN UND POLYHARNSTOFFEN ALS HILFSMITTEL IN KOSMETISCHEN UND PHARMAZEUTISCHEN ZUBEREITUNGEN**
USE OF CATIONIC POLYURETHANES AND POLYCARBAMIDES AS AUXILIARY SUBSTANCES IN COSMETIC AND PHARMACEUTICAL COMPOSITIONS
UTILISATION DE POLYURETHANNES ET DE POLYCARBAMIDES CATIONIQUES COMME ADJUVANTS DANS DES COMPOSITIONS COSMETIQUES ET PHARMACEUTIQUES

(30) Priorität: 07.12.1992 DE 4241118
(43) Veröffentlichungstag der Anmeldung: 20.09.1995
(73) Patentinhaber: BASF Aktiengesellschaft, 67063 Ludwigshafen (DE)
(72) Erfinder: NGUYEN KIM, Son, D-69502 Hemsbach (DE); SANNER, Axel, D-67227 Frankenthal (DE); SPERLING-VIETMEIER, Karin, D-67433 Neustadt (DE); HOESSEL, Peter, D-67105 Schifferstadt (DE)
(86) Internationale Anmeldenummer: EP9303306
(87) Internationale Veröffentlichungsnummer: WO9413724

(56) Entgegenhaltungen:
- EP-A- 0 433 776
- DE-B- 2 019 324
- FR-A- 2 162 025

## Beschreibung

Die vorliegende Erfindung betrifft die Verwendung von kationischen Polyurethanen und Polyharnstoffen als Hilfsmittel in kosmetischen und pharmazeutischen Zubereitungen. Da ein Teil dieser Verbindungen neue Stoffe sind, betrifft die Erfindung weiterhin diese neuen Polyurethane und Polyharnstoffe.

Polyurethane und Polyharnstoffe, die kationische Gruppen durch Einbau von quaternierbaren oder protonierbaren tertiären Aminstickstoffatomen enthalten, sind bereits bekannt. So werden beispielsweise in der DE-A 20 19 324 (1) lichtechte Polyurethanionomere mit tertiärem oder quartärem Ammoniumstickstoff beschrieben, welche Struktureinheiten der Formel 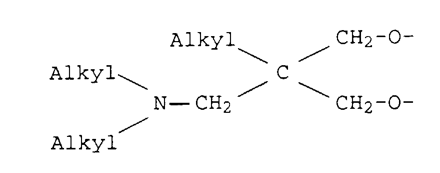 aufweisen, wobei das Stickstoffatom quaternisiert oder protoniert vorliegen kann. Diese Polyurethanionomere werden für die verschiedenartigsten Anwendungsgebiete empfohlen, der Kosmetik- und der Pharmabereich werden jedoch nicht erwähnt.

Aus der DE-C 11 78 586 (2) sind Polyurethane auf der Basis von Polyhydroxylverbindungen mit einem Molekulargewicht von 400 bis 10.000 und Polyisocyanaten bekannt, wobei mindestens eine der Komponenten mindestens ein basisches tertiäres Aminstickstoffatom enthält. So wird in Beispiel 14 die Herstellung eines Polyurethans aus einem Adipinsäure-Hexandiol-Polyester, Toluylendiisocyanat, 1,4-Butandiol und einer geringen Menge an N-Methyldiethanolamin beschrieben. Für das Polyurethan aus Beispiel 14 errechnet sich eine Aminzahl von 27. Diese Polyurethane werden unter anderem auch als Haarfixiermittel empfohlen.

FR 2 162 025 beschreibt kationische Polykondensationsprodukte auf Piperazinbasis als Haarbehandlungsmittel.

In der Kosmetik werden Haarbehandlungsmittel, die beispielsweise als Haarverfestiger oder Haarspray vorliegen, zum Festigen, Strukturverbessern und Formgeben der Haare verwendet. Die Haarbehandlungsmittel bestehen vorwiegend aus einer Lösung von filmbildenden Harzen oder synthetischen Polymeren. Bisher wurden in Haarbehandlungsmitteln hauptsächlich folgende Filmbildner verwendet: Schellack, Homo- und Copolymerisate des N-Vinylpyrrolidons, Copolymerisate von Vinylethern/Maleinsäurehalbestern, von (Meth)acrylsäure oder deren Estern und Amiden und Crotonsäure mit Vinylestern. in Haarbehandlungsmitteln hauptsächlich folgende Filmbildner verwendet: Schellack, Homo- und Copolymerisate des N-Vinylpyrrolidons, Copolymerisate von Vinylethern/Maleinsäurehalbestern, von (Meth)acrylsäure oder deren Estern und Amiden und Crotonsäure mit Vinylestern.

Die Haarbehandlungsmittel werden in Form von Lösungen, vorzugsweise als ethanolische Lösungen, durch Sprühen auf die Haare gebracht. Nach dem Verdampfen des Lösemittels werden die Haare an den gegenseitigen Berührungspunkten vom zurückbleibenden Polymer in der gewünschten Form gehalten. Die Polymeren sollen einerseits so hydrophil sein, daß sie aus dem Haar ausgewaschen werden können, andererseits sollen sie hydrophob sein, damit die mit den Polymeren behandelten Haare auch bei hoher Luftfeuchtigkeit ihre Form behalten und nicht miteinander verkleben.

Die bisher bekannten polymeren Filmbildner wie Polyvinylpyrrolidone zeigen jedoch meistens als Nachteil eine zu hohe Wasseraufnahme bei erhöhter Luftfeuchtigkeit. Diese Eigenschaft führt u.a. zu einem unerwünschten Verkleben der Haare und zu einem Verlust der Festigkeit und damit einem Zusammenbruch der Haarfrisur. Wird andererseits die Widerstandsfähigkeit gegen hohe Luftfeuchtigkeit verbessert, z.B. bei Copolymerisaten aus N-Vinylpyrrolidon und Vinylacetat, so leidet darunter die Elastizität des Films und die Sprödigkeit dieser Filme kann nach der Haarbehandlung sogar zu einem unangenehmen Stauben und einem schuppigen Belag führen. Außerdem wird vor allem die Auswaschbarkeit bei der Reinigung der Haare sehr erschwert. Die obengenannten synthetischen Haarbehandlungsmittel sind aufgrund ihrer hydrolysebeständigen C-C-Kette biologisch nicht abbaubar. Schellack ist dagegen biologisch abbaubar, hat aber viele Nachteile. So sind seine Eigenschaften als Haarbehandlungsmittel im Vergleich zu den Homo- und Copolymerisaten des N-Vinylpyrrolidons schlechter, insbesondere bezüglich der Klebrigkeit, Wasserlöslichkeit und Steifigkeit. Da Schellack ein Naturprodukt ist, sind seine Eigenschaften starken Schwankungen unterlegen.

Der vorliegenden Erfindung lag daher die Aufgabe zugrunde, Hilfsmittel für kosmetische und pharmazeutische Zubereitungen bereitzustellen, die die geschilderten Nachteile des Standes der Technik nicht mehr aufweisen.

Demgemäß wurde die Verwendung von kationischen Polyurethanen und Polyharnstoffen aus
(a) mindestens einem Diisocyanat, welches bereits vorher mit einer oder mehreren Verbindungen, die zwei oder mehrere aktive Wasserstoffatome pro Molekül enthalten, umgesetzt sein kann, und
(b) mindestens einem ein oder mehrere tertiäre, quartare oder protonierte tertiäre Aminstickstoffatome enthaltenden Diol, primären oder sekundären Aminoalkohol, primären oder sekundären Diamin oder primären oder sekundären Triamin
mit einer Glasübergangstemperatur von mindestens 25°C und einer Aminzahl von 50 bis 200, bezogen auf die nicht quaternisierten oder protonierten Verbindungen, oder sonstigen Salzen dieser Polyurethane und Polyharnstoffe als Hilfsmittel in kosmetischen und pharmazeutischen Zubereitungen gefunden.

Als Verbindungen der Gruppe (a) kommen insbesondere C₂- bis C₈-Alkylendiisocyanate, z.B. 1,2-Ethylendiisocyanat, 1,4-Butylendiisocyanat, Hexamethylendiisocyanat oder Octamethylendiisocyanat, C₅- bis C₁₀-Cycloalkylendiisocyanate, z.B. 1,3-Cyclopentylendiisocyanat, 1,3- oder 1,4-Cyclohexylendiisocyanat oder Isophorondiisocyanat, o-, m- oder p-Phenylendiisocyanat oder (C₁- bis C₄-Alkyl)phenylendiisocyanate, z.B. Toluylendiisocyanat, in Betracht. Diese Diisocyanate können bereits vorher mit einer oder mehreren Verbindungen aus der Gruppe der Diole, Aminoalkohole, Diamine, Polyesterole, Polyamiddiamine und Polyetherole mit einem zahlengemittelten Molekulargewicht von jeweils bis zu 2000 umgesetzt sein, wobei bis zu 3 mol-% der letztgenannten Verbindungen durch Triole oder Triamine ersetzt sein und die Diole und Aminoalkohle ein oder mehrere tertiäre, quartäre oder protonierte tertiäre Aminstickstoffatome enthalten können.

Geeignete Diole sind beispielsweise Ethylenglykol, Propylenglykol, Butylenglykol, Neopentylglykol, Polyetherole wie Polyethylenglykole, Polypropylenglykole oder Polytetrahydrofurane, Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Vorzugsweise verwendet man aus der Gruppe der Diole und Polyetherole Ethylenglykol, Neopentylglykol, Diethylenglykol, Triethylenglykol, Tetraethylenglykol, Pentaethylenglykol und Hexaethylenglykol.

Geeignete Aminoalkohole sind beispielsweise 2-Aminoethanol, 2-(N-Methylamino)ethanol, 3-Aminopropanol oder 4-Aminobutanol.

Geeignete Diamine sind beispielsweise Ethylendiamin, Propylendiamin, 1,4-Diaminobutan und Hexamethylendiamin-1,6 sowie α,ω-Diamine, die durch Aminierung von Polyalkylenoxiden, insbesondere Polyethylenoxiden mit Ammoniak herstellbar sind.

Als Polyesterole kommen solche in Betracht, die üblicherweise zur Herstellung von Polyurethanen eingesetzt werden, z.B. Umsetzungsprodukte aus Phthalsäure und Diethylenglykol, Isophthalsäure und Butandiol-(1,4), Isophthalsäure/Adipinsäure und Hexandiol-(1,6) sowie aus Adipinsäure und Ethylenglykol.

Zur Herstellung der Vorprodukte aus den Diisocyanaten und den Verbindungen mit aktiven Wasserstoffatomen kann man auch Mischungen dieser Verbindungen einsetzen, z.B. Mischungen aus einem Diol und einem Polyesterol, oder einem Diol und Polyetherolen. In den Mischungen können bis zu 3 mol-% der genannten Verbindungen durch Triole oder Triamine ersetzt sein. Geeignete Triole sind beispielsweise Glycerin, Trimethylolethan oder Trimethylolpropan. Als Triamine eignen sich insbesondere Diethylentriamin oder Dipropylentriamin.

In einer bevorzugten Ausführungsform setzt man zur Herstellung der Vorprodukte als Verbindungen mit aktiven Wasserstoffatomen mindestens 5 mol-% eines Poly(milchsäureesterdiols) der allgemeinen Formel I 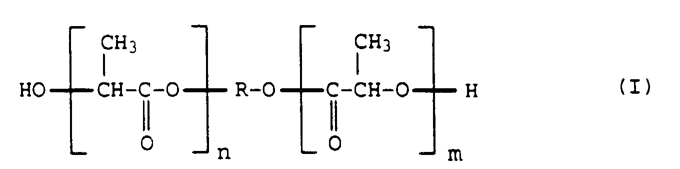 eines Poly(ε-caprolactondiols) der allgemeinen Formel II 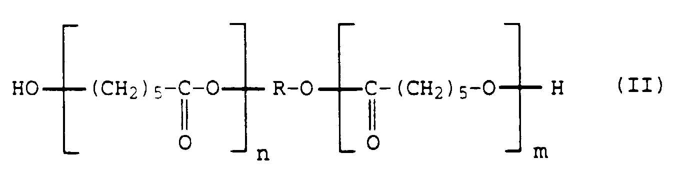 oder eines Polyamiddiamins der allgemeinen Formel III 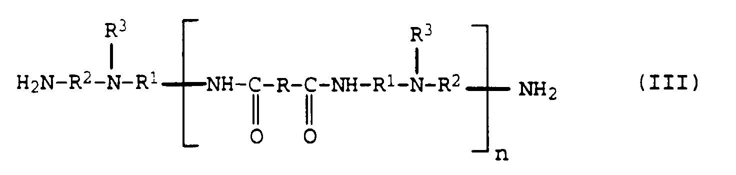 in denen
- R: C₂- bis C₈-Alkylen, C₅- bis C₈-Cycloalkylen oder Phenylen bezeichnet,
- R¹ und R²: C₂- bis C₈-Alkylen bedeuten,
- R³: für C₁- bis C₄-Alkyl, Phenyl oder C₇- bis C₁₀-Phenylalkyl steht und
- n und m: jeweils eine Zahl von 1 bis 30 bezeichnet,
ein.

Als C₂- bis C₈-Alkylengruppen für R, R¹ und R² kommen vor allem 1,2-Ethylen, 1,3-Propylen, 1,4-Butylen und 2,2-Dimethyl-1,3-propylen, daneben aber auch 1,2-Propylen, 1,2-Butylen, 2,3-Butylen, Pentamethylen, Hexamethylen, Heptamethylen oder Octamethylen in Betracht.

Als C₅- bis C₈-Cycloalkylengruppen für R eignen sich vor allem die Gruppierung der Formel 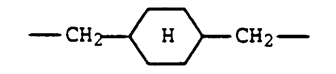 daneben aber auch 1,3-Cyclopentylen, 1,3-Cyclohexylen, 1,4-Cyclohexylen, 1,4-Cycloheptylen oder Gruppierungen der Formeln 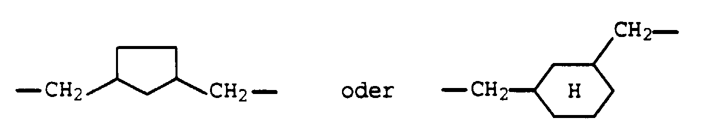 Als Phenylengruppe für R eignen sich o-, m- und vor allem p-Phenylen.

Als C₁- bis C₄-Alkylreste für R³ und auf für die (C₁- bis C₄-Alkyl)phenylendiisocyanate kommen vor allem Methyl und Ethyl, daneben aber auch n-Propyl, iso-Propyl, n-Butyl, iso-Butyl, sec.-Butyl und tert.-Butyl in Betracht.

Als C₇- bis C₁₀-Phenylalkyl eignen sich insbesondere Benzyl und 2-Phenylethyl, daneben aber auch o-, m- und p-Methylbenzyl, 3-Phenylpropyl und 4-Phenylbutyl.

n und m bezeichnen vorzugsweise jeweils eine Zahl von 1 bis 15, insbesondere von 1 bis 7.

Ein gut geeignetes Beispiel für ein Polyamiddiamin III ist das Kondensationsprodukt aus k mol Adipinsäure und (k+1) mol N-Methyldipropylentriamin, wobei k für eine Zahl von 2 bis 5 steht.

Die Verbindungen der Gruppe (b) enthalten vorzugsweise Diole, Aminoalkohole, Diamine oder Triamine mit quartären oder protonierten Aminstickstoffatomen, da geladene N-Atome die Löslichkeit der erfindungsgemäß anzuwendenden Polyharnstoffe stark erhöhen.

In einer bevorzugten Ausführungsform setzt man als Verbindungen der Gruppe (b) eine oder mehrere Verbindungen der allgemeinen Formeln IV bis XI 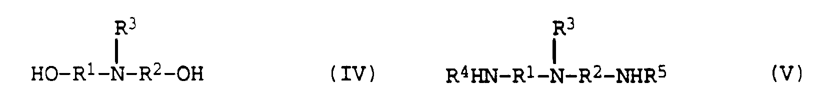 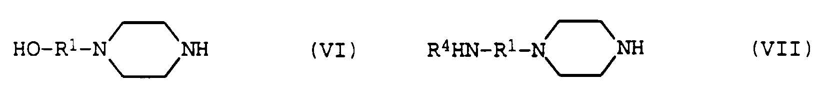 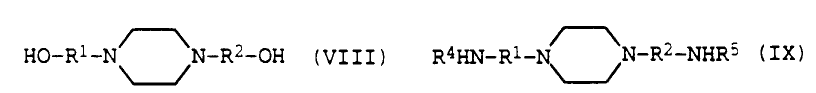 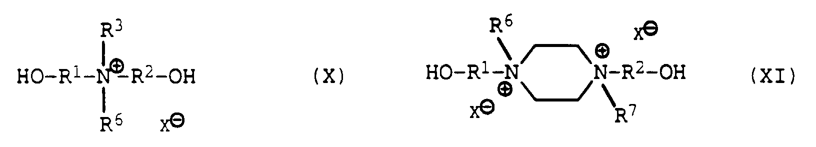 in denen
- R¹ und R²: C₂- bis C₈-Alkylen bedeuten,
- R¹ , R⁶ und R⁷: für C₁- bis C₄-Alkyl, Phenyl oder C₇- bis C₁₀-Phenylalkyl stehen,
- R⁴ und R⁵: Wasserstoff oder C₁- bis C₄-Alkyl bezeichnen und
- X^{⊖}: für Chlorid, Bromid, Iodid, C₁- bis C₄-Alkylsulfat oder die halbe stöchiometrische Menge Sulfat steht,
ein. Dabei werden die Polyurethane bzw. Polyharnstoffe vor der erfindungsgemäßen Verwendung zweckmäßigerweise quaternisiert oder protoniert, wenn nicht schon eine quaternisierte oder protonierte Verbindung (b), z.B. X oder XI, verwendet wurde.

Für die einzelnen Bedeutungen von C₂- bis C₈-Alkylen, C₁- bis C₄-Alkyl und C₇- bis C₁₀-Phenylalkyl gilt das oben gesagte.

Wie bei der Herstellung von Polyurethanen und Polyharnstoffen üblich, kann man Kettenverlängerer verwenden. Geeignete Kettenverlängerer sind beispielsweise Hexamethylendiamin, Piperazin, 1,2-Diaminocyclohexan, 1,3-Diaminocyclohexan, 1,4-Diaminocyclohexan, Neopentandiamin und 4,4'-Diaminodicyclohexylmethan.

Die beschriebenen Polyurethane und Polyharnstoffe sind vorzugsweise dadurch erhältlich, daß man die Reaktionspartner für die Diisocyanate unter einer Inertgasatmosphäre in einem inerten Lösemittel, z.B. Methylethylketon bei Verbindung mit OH-Gruppen und Wasser oder einem Alkohol wie Ethanol bei Verbindungen mit NH-Gruppen, bei Temperaturen von 50 bis 130°C bei Verbindungen mit OH-Gruppen und 5 bis 30°C bei Verbindungen mit NH-Gruppen mit den Diisocyanaten umsetzt. Diese Umsetzung kann gegebenenfalls in Gegenwart von Kettenverlängerern durchgeführt werden, um Polyurethane bzw. Polyharnstoffe mit höheren Molekulargewichten herzustellen. Die Umsetzung kann durch Zugabe von Katalysatoren wie zinnorganischen Verbindungen, z.B. Dibutylzinndilaurat, insbesondere bei Reaktanden mit OH-Gruppen, beschleunigt werden. Wie bei der Herstellung von Polyurethanen üblich, werden die Reaktionspartner für die Diisocyanate und die Diisocyanate selbst zweckmäßigerweise im molaren Verhältnis von 0,8 bis 1,1 : 1 eingesetzt.

Die Aminzahl der Polyurethane bzw. Polyharnstoffe wird von der Zusammensetzung, insbesondere vom Anteil der Verbindungen mit tertiären, quartären oder protonierten tertiären Aminstickstoffatomen in der Mischung bestimmt. Die Aminzahl liegt vorzugsweise bei 65 bis 180, insbesondere bei 70 bis 170, besonders bevorzugt bei 75 bis 160 und ganz besonders bevorzugt bei 80 bis 150.

Bei der Herstellung der beschriebenen Polyurethane und Polyharnstoffe beträgt der Anteil der Verbindungen mit NH-Gruppen, bezogen auf die Menge der Verbindungen mit OH-Gruppen, in der Regel 35 bis 100 Gew.-%, vorzugsweise 40 bis 100 Gew.-%, insbesondere 50 bis 100 Gew.-%.

Die Polyurethane und Polyharnstoffe haben üblicherweise K-Werte nach H. Fikentscher (bestimmt in 0,1 gew.-%igen Lösungen in N-Methylpyrrolidon bei 25°C und pH 7) von 15 bis 100, vorzugsweise 20 bis 50.

Die Glasübergangstemperatur der beschriebenen Polyurethane und Polyharnstoffe liegt üblicherweise bei 25 bis 140°C, wobei unter 25°C die Polyharnstoffe keine ausreichenden Filmbildungseigenschaften mehr aufweisen. Ein bevorzugter Bereich ist 50 bis 120°C. Die Glasübergangstemperatur kann nach ASTM D 3418 bestimmt werden.

Die beschriebenen Polyurethane und Polyharnstoffe sind aufgrund ihrer kationischen Gruppierungen, insbesondere beim Vorliegen von Ladungen, in der Regel leicht alkohol- und wasserlöslich oder zumindest ohne Zuhilfenahme von Emulgatoren in Alkohol und Wasser dispergierbar. Als Alkohole sind hier insbesondere kurzkettige Alkanole wie Methanol, Ethanol, iso-Propanol oder n-Propanol gemeint. Geladene kationische Gruppierungen lassen sich aus den vorliegenden tertiären Aminstickstoffatomen entweder durch Protonierung, z.B. mit Carbonsäuren wie Milchsäure, oder durch Quaternisierung, z.B. mit Alkylierungsmitteln wie C₁- bis C₄-Alkylhalogeniden oder -sulfaten, in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Da ein Teil der beschriebenen Polyurethane und Polyharnstoffe neue Stoffe darstellen, betrifft die vorliegende Erfindung weiterhin diese neuen Stoffe.

Demgemäß sind Gegenstand der vorliegenden Erfindung Polyurethane und Polyharnstoffe aus
(a) C₂- bis C₈-Alkylendiisocyanaten, C₅- bis C₁₀-CYcloalkylendiisocyanaten, Phenylendiisocyanaten oder (C₁- bis C₄-Alkyl)phenylendiisocyanaten, welche bereits vorher mit einer oder mehreren Verbindungen aus der Gruppe der Diole, Aminoalkohole, Diamine, Polyesterole, Polyamiddiamine und Polyetherole mit einem zahlengemittelten Molekulargewicht von jeweils bis zu 2000 umgesetzt sein können, wobei bis zu 3 mol-% der letztgenannten Verbindungen durch Triole oder Triamine ersetzt sein können, die Diole und Aminoalkohole ein oder mehrere tertiäre, quartäre oder protonierte tertiäre Aminstickstoffatome enthalten können und wobei mindestens 5 mol-% der mit den Diisocyanaten bereits vorher umgesetzten Verbindungen ein Poly(milchsäureesterdiol) der allgemeinen Formel I 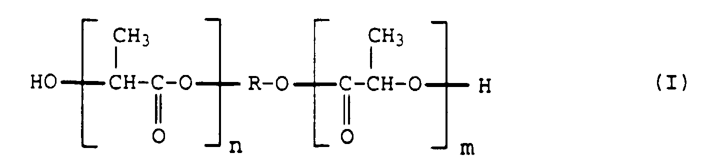 ein Poly(ε-caprolactondiol) der allgemeinen Formel II 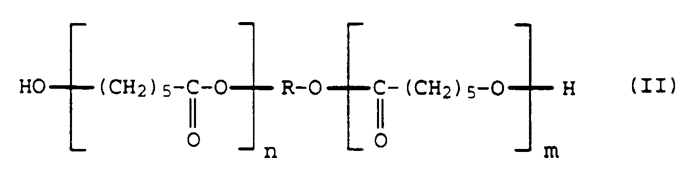 oder ein Polyamiddiamin der allgemeinen Formel III 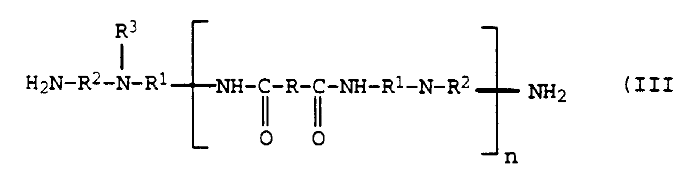 in denen
   - R: C₂- bis C₈-Alkylen, C₅- bis C₈-Cycloalkylen oder Phenylen bezeichnet,
   - R¹ und R²: C₂- bis C₈-Alkylen bedeuten,
   - R³: für C₁- bis C₄-Alkyl, Phenyl oder C₇- bis C₁₀-Phenylalkyl steht und
   - n und m: jeweils eine Zahl von 1 bis 30 bezeichnet,
   darstellen, und
(b) mindestens einem ein oder mehrere tertiäre, quartäre oder protonierte tertiäre Aminstickstoffatome enthaltenden Diol, primären oder sekundären Aminoalkoholen, primären oder sekundären Diamin oder primären oder sekundären Triamin mit einer Glasübergangstemperatur von mindestens 25°C und einer Aminzahl von 50 bis 200, bezogen auf die nicht quaternisierten oder protonierten Verbindungen, und sonstige Salze dieser Polyurethane und Polyharnstoffe.

Insbesondere sind Gegenstand der vorliegenden Erfindung solche Polyurethane und Polyharnstoffe der oben bezeichneten Zusammensetzung, bei denen als Verbindungen der Gruppe (b) eine oder mehrere Verbindungen der allgemeinen Formeln IV bis XI 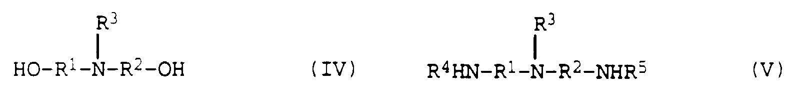 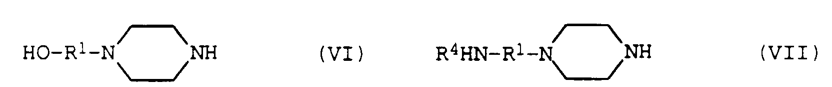 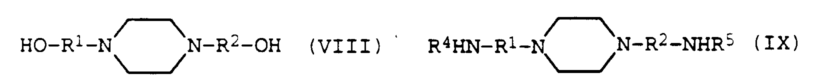 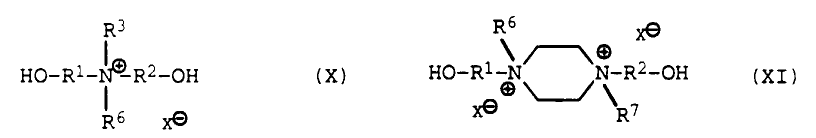 in denen
- R¹ und R²: C₂- bis C₈-Alkylen bedeuten,
- R¹, R⁶ und R⁷: für C₁- bis C₄-Alkyl, Phenyl oder C₇- bis C₁₀-Phenylalkyl stehen,
- R⁴ und R⁵: Wasserstoff oder C₁- bis C₄-Alkyl bezeichnen und
- X^{⊖}: für Chlorid, Bromid, Iodid, C₁- bis C₄-Alkylsulfat oder die halbe stöchiometrische Menge Sulfat steht,
eingesetzt werden.

Die beschriebenen Polyharnstoffe sind in der Regel zumindest teilweise biologisch abbaubar.

Die beschriebenen Polyurethane und Polyharnstoffe werden außer in der Haarkosmetik als Filmbildner in Sprays, Schäumen, Festiger oder Gelen oder als Konditioner in Haarpflegespülungen oder Shampoos auch für Cremes und im Pharmabereich als Tablettenüberzugsmittel und Tablettenbinder verwendet.

Gegenstand der vorliegenden Erfindung sind auch kosmetische und pharmazeutische Zubereitungen, welche als Hilfsmittel in wirksamen Mengen die beschriebenen kationischen Polyurethane oder Polyharnstoffe oder sonstige Salze hiervon enthalten. Als wirksame Menge sind in der Regel, je nach Anwendung, 0,1 bis 50 Gew.-%, insbesondere 0,5 bis 30 Gew.-%, bezogen auf die Zubereitung, anzusehen.

Sofern die beschriebenen Polyurethane und Polyharnstoffe als Haarbehandlungsmittel mit filmbildenden Eigenschaften verwendet werden, gelangen sie meistens in Form von wäßrigen oder ethanolischen Lösungen zur Anwendung. Der Feststoffgehalt dieser Lösungen beträgt 0,1 bis 30, vorzugsweise 1 bis 15 Gew.-% Polyurethan bzw. Polyharnstoff oder eines Salzes hiervon.

### Beispiele

### Allgemeine Herstellungsvorschrift

In einem Vierhalskolben, der mit Rührer, Tropftrichter, Thermometer, Rückflußkühler und Vorrichtung für das Arbeiten unter Stickstoff ausgestattet ist, werden die in der Tabelle angegebenen Verbindungen mit OH-Gruppen im Methylethylketon gelost. Dazu wird das Reaktionsgemisch auf eine Temperatur von ca. 80°C unter Rühren erhitzt. Sobald sich alles gelöst hat, kühlt man das Reaktionsgemisch auf ca. 60°C ab und tropft unter Rühren das in der Tabelle jeweils angegebene Diisocyanat zu. Die Reaktionstemperatur steigt dabei an. Bei einer Innentemperatur von 90°C wird das Reaktionsgemisch dann solange gerührt, bis der Isocyanatgruppengehalt des Gemisches praktisch konstant bleibt. Danach kühlt man das Reaktionsgemisch auf eine Temperatur in dem Bereich von 10°C bis 20°C ab, gibt Ethanol zu und tropft bei dieser Temperatur die in der Tabelle angegebenen Verbindungen mit NH-Gruppen und gegebenenfalls Kettenverlängerer mit NH-Gruppen langsam zu. Man rührt das Reaktionsgemisch dann noch solange in diesem Temperaturbereich, bis der Isocyanatgruppengehalt auf einen konstanten Wert abgefallen ist. Sofern man keinen Kettenverlängerer zugesetzt hat, werden die restlichen Isocyanatgruppen durch Zusatz von Aminen, z.B. 2-Amino-2-methyl-1-propanol, inaktiviert. Durch Zugabe eines Protonierungs- bzw. Quaternisierungsmittels gemäß Tabelle wird das Endprodukt hergestellt. Man destilliert den größten Teil des Methylethylketons und des Ethanols unter vermindertem Druck bei ca. 40°C ab. Das restliche Ethanol wird im Vakuumtrockenschrank bei 50°C entfernt. Man erhält nach dem Trocknen ein elastisches bis sehr hartes Produkt, das in Ethanol sowie in Wasser löslich bzw. dispergierbar ist.

Setzt man nur NH-Gruppen-haltige Verbindungen mit dem Diisocyanat um, arbeitet man gleich bei 10°C bis 20°C in Ethanol ohne Methylethylketon.

Anstelle von Ethanol kann man auch Wasser verwenden. Das als Lösemittel verwendete Methylethylketon oder Ethanol kann dann nach erfolgter Umsetzung im Vakuum bei 40°C abdestilliert werden, so daß man direkt eine wäßrige Lösung bzw. Dispersion des Polyharnstoffs mit den in der Tabelle angegebenen Eigenschaften erhält.

Die Abkürzungen in der Tabelle haben folgende Bedeutung:

| | |
|---|---|
| MDEA | N-Methyldiethanolamin |
| MDPTA | N-Methyldipropylentriamin |
| AEP | 2-Aminoethylpiperazin |
| P(MIS-EG) | Polymilchsäure-ethylenglykol (M_{w}= 500 g/mol) |
| P(IPS/ADS-VI) | Polyesterol aus Isophthalsäure, Adipinsäure und Hexandiol-(1,6) (M_{w}=1000g/mol) |
| NPG | Neopentylglykol |
| IPDI | Isophorondiisocyanat |
| MIS | Milchsäure |
| DES | Diethylsulfat |
| NMP | N-Methylpyrrolidon |
| 1 | leicht löslich |
| disp | dispergierbar |
| | |

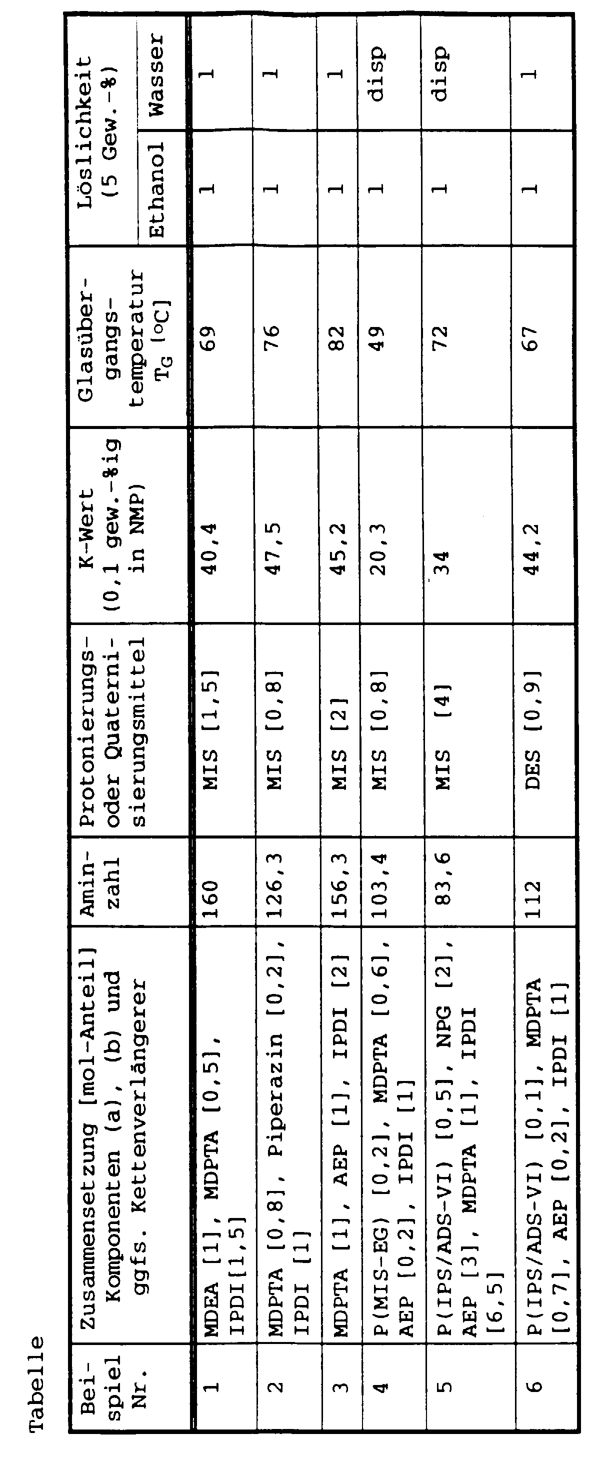

Um die Verwendung als Haarbehandlungsmittel zu demonstrieren, wurden folgende Formulierungen hergestellt:
A) Aerosol-Haarspray (rein ethanolisch)

| | |
|---|---|
| Produkt gemäß Beispiel 5 | 2 Gew.-% |
| Ethanol abs. | 73 Gew.-% |
| Dimethylether | 25 Gew.-% |

B) Aerosol-Haarspray (wäßrig-ethanolisch)

| | |
|---|---|
| Produkt gemäß Beispiel 5 | 3 Gew.-% |
| Wasser dest. | 12 Gew.-% |
| Ethanol abs. | 60 Gew.-% |
| Dimethylether | 25 Gew.-% |

C) Haarfestigerlösung (wäßrig-ethanolisch)

| | |
|---|---|
| Produkt gemäß Beispiel 5 | 4 Gew.-% |
| Wasser dest. | 64 Gew.-% |
| Ethanol abs. | 32 Gew.-% |

Mit der Formulierung A nach der üblichen Methode behandeltes Haar wies einen Curl-Retention-Wert von 92 % und eine Biegesteifigkeit von 129 p auf. In analoger Weise mit handelsüblichen Haarfixiermitteln behandeltes Haar wiesen entsprechende Werte für die Curl Retention von 35 % (mit N-Vinylpyrrolidon-Vinylacetat-Haarpolymer) und 90 % (mit N-Vinylpyrrolidon-tert.-Butylacrylat-Acrylsäure-Haarpolymer) und für die Biegesteifigkeit von 59 p bzw. 69 p auf.

Der als Maß für die Festigungswirkung (engl. stiffness) durchgeführte Biegesteifigkeitstest wurde gemäß der Literaturstelle Parfums, cosmetiques, arômes n° 89, Octobre-Novembre 1989, 71, vorgenommen. Er wurde auch auf dem BASF-Symposium "Cosmeticon" am 10.-11. Mai 1990 in Heidelberg vorgestellt. Dieser Test gibt an, welche Kraft nötig ist, um eine mit der filmbildenden Polymerlösung behandelte Haarsträhne bis zum Bruch der Filmhaut durchzubiegen. Je größer die Kraft ist, desto höher ist die Festungswirkung.

## Patentansprüche

1. Verwendung von kationischen Polyurethanen und Polyharnstoffen aus
(a) mindestens einem Diisocyanat, welches bereits vorher mit einer oder mehreren Verbindungen, die zwei oder mehrere aktive Wasserstoffatome pro Molekül enthalten, umgesetzt sein kann, und
(b) mindestens einem ein oder mehrere tertiäre, quartäre oder protonierte tertiäre Aminstickstoffatome enthaltenden Diol, primären oder sekundären Aminoalkohol, primären oder sekundären Diamin oder primären oder sekundären Triamin
mit einer Glasübergangstemperatur von mindestens 25°C und einer Aminzahl von 50 bis 200, bezogen auf die nicht quaternisierten oder protonierten Verbindungen, oder sonstigen Salzen dieser Polyurethane und Polyharnstoffe als Hilfsmittel in kosmetischen und pharmazeutischen Zubereitungen.

2. Verwendung nach Anspruch 1, wobei als Verbindungen der Gruppe (a) C₂- bis C₈-Alkylendiisocyanate, C₅- bis C₁₀-Cycloalkylendiisocyanate, Phenylendiisocyanate oder (C₁- bis C₄-Alkyl)phenylendiisocyanate eingesetzt werden, welche bereits vorher mit einer der mehreren Verbindungen aus der Gruppe der Diole, Aminoalkohole, Diamine, Polyesterole, Polyamiddiamine und Polyetherole mit einem zahlengemittelten Molekulargewicht von jeweils bis zu 2000 umgesetzt sein können, wobei bis zu 3 mol-% der letztgenannten Verbindungen durch Triole oder Triamine ersetzt sein und die Diole und Aminoalkohole ein oder mehrere tertiäre, quartäre oder protonierte tertiäre Aminstickstoffatome enthalten können.

3. Verwendung nach Anspruch 1 oder 2, wobei als mit den Diisocyanaten bereits vorher umgesetzte Verbindungen mindestens 5 mol-% eines Poly(milchsäureesterdiols) der allgemeinen Formel I 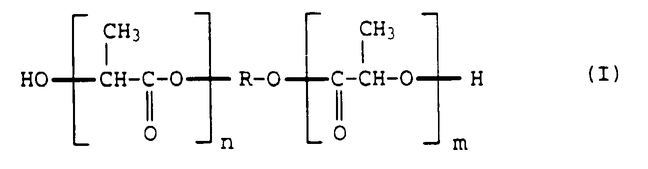 eines Poly(ε-caprolactondiols) der allgemeinen Formel II 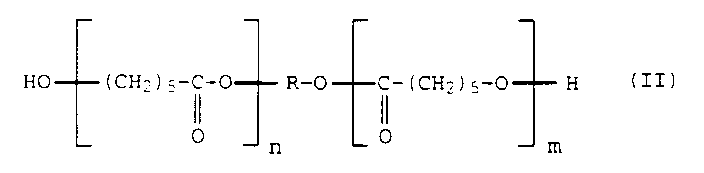 oder eines Polyamiddiamins der allgemeinen Formel III 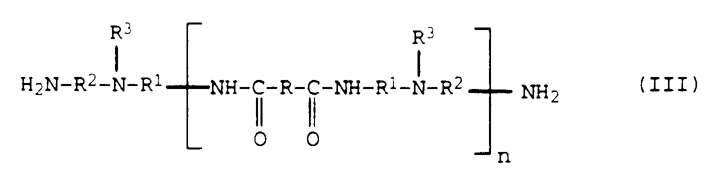 in denen
R C₂- bis C₈-Alkylen, C₅- bis C₈-Cycloalkylen oder Phenylen bezeichnet,
R¹ und R² C₂- bis C₈-Alkylen bedeuten,
R³ für C₁- bis C₄-Alkyl, Phenyl oder C₇- bis C₁₀-Phenylalkyl steht und
n und m jeweils eine Zahl von 1 bis 30 bezeichnet,
eingesetzt werden.

4. Verwendung nach Anspruch 1, wobei als Verbindungen der Gruppe (b) eine oder mehrere Verbindungen der allgemeinen Formeln IV bis XI 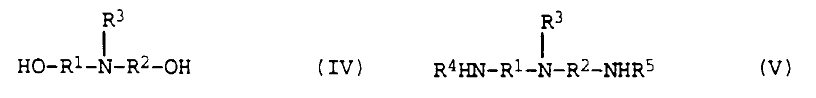 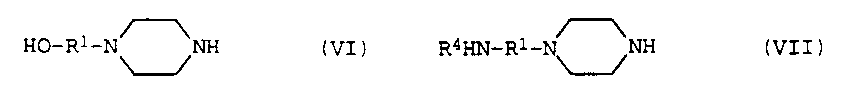 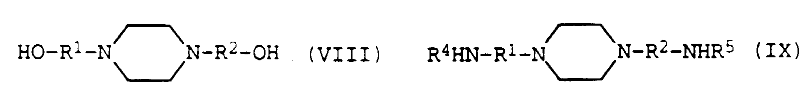 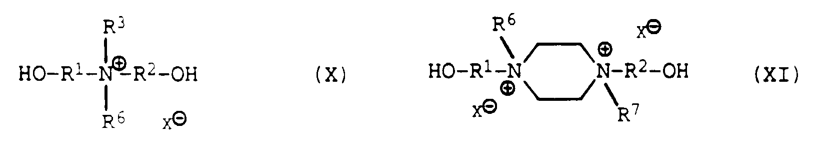 in denen
R¹ und R² C₂- bis C₈-Alkylen bedeuten,
R³, R⁶ und R⁷ für C₁- bis C₄-Alkyl, Phenyl oder C₇- bis C₁₀-Phenylalkyl stehen,
R⁴ und R⁵ Wasserstoff oder C₁- bis C₄-Alkyl bezeichnen und
X^{⊖} für Chlorid, Bromid, Iodid, C₁- bis C₄-Alkylsulfat oder die halbe stöchiometrische Menge Sulfat steht,
eingesetzt werden.

5. Kosmetische und pharmazeutische Zubereitungen, enthaltend als Hilfsmittel in wirksamen Mengen kationische Polyurethane oder Polyharnstoffe aus
(a) mindestens einem Diisocyanat, welches bereits vorher mit einer oder mehreren Verbindungen, die zwei oder mehrere aktive Wasserstoffatome pro Molekül enthalten, umgesetzt sein kann, und
(b) mindestens einem ein oder mehrere tertiäre, quartäre oder protonierte tertiäre Aminstickstoffatome enthaltenden Diol, primären oder sekundären Aminoalkohol, primären oder sekundären Diamin oder primären oder sekundären Triamin
mit einer Glasübergangstemperatur von mindestens 25°C und einer Aminzahl von 50 bis 200, bezogen auf die nicht quaternisierten oder protonierten Verbindungen, oder sonstige Salze dieser Polyurethane und Polyharnstoffe.

6. Kationische Polyurethane und Polyharnstoffe aus
(a) C₂- bis C₈-Alkylendiisocyanaten, C₅- bis C₁₀-Cycloalkylendiisocyanaten, Phenylendiisocyanaten oder (C₁- bis C₄-Alkyl)phenylendiisocyanaten, welche bereits vorher mit einer oder mehreren Verbindungen aus der Gruppe der Diole, Aminoalkohole, Diamine, Polyesterole, Polyamiddiamine und Polyetherole mit einem zahlengemittelten Molekulargewicht von jeweils bis zu 2000 umgesetzt sein können, wobei bis zu 3 mol-% der letztgenannten Verbindungen durch Triole oder Triamine ersetzt sein können, die Diole und Aminoalkohole ein oder mehrere tertiäre, quartäre oder protonierte tertiäre Aminstickstoffatome enthalten können und wobei mindestens 5 mol-% der mit den Diisocyanaten bereits vorher umgesetzten Verbindungen ein Poly(milchsaureesterdiol) der allgemeinen Formel I 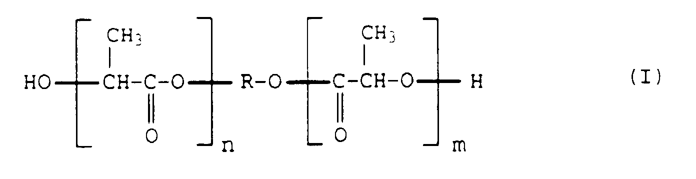 ein Poly(ε-caprolactondiol) der allgemeinen Formel II 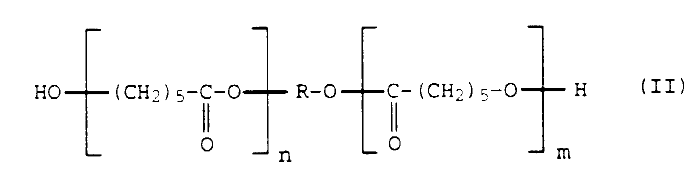 oder ein Polyamiddiamin der allgemeinen Formel III 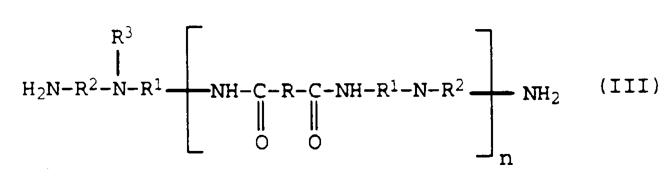 in denen
R C₂- bis C₈-Alkylen, C₅- bis C₈-Cycloalkylen oder Phenylen bezeichnet,
R¹ und R² C₂- bis C₈-Alkylen bedeuten,
R³ für C₁- bis C₄-Alkyl, Phenyl oder C₇- bis C₁₀-Phenylalkyl steht und
n und m jeweils eine Zahl von 1 bis 30 bezeichnet,
darstellen, und
(b) mindestens einem ein oder mehrere tertiäre, quartäre oder protonierte tertiäre Aminstickstoffatome enthaltenden Diol, primären oder sekundären Aminoalkohol, primären oder sekundären Diamin oder primären oder sekundären Triamin mit einer Glasübergangstemperatur von mindestens 25°C und einer Aminzahl von 50 bis 200, bezogen auf die nicht quaternisierten oder protonierten Verbindungen, und sonstige Salze dieser Polyurethane und Polyharnstoffe.

7. Kationische Polyurethane und Polyharnstoffe nach Anspruch 6, wobei als Verbindungen der Gruppe (b) eine oder mehrere Verbindungen der allgemeinen Formeln IV bis XI 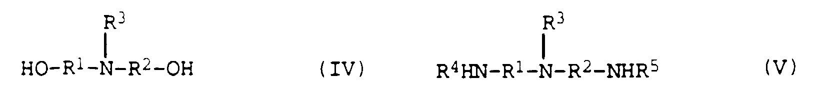 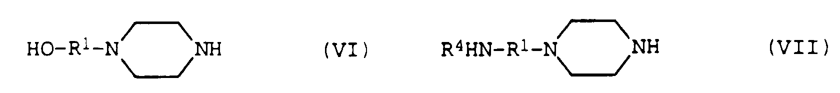 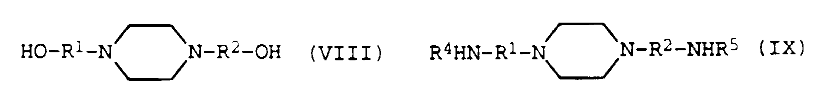 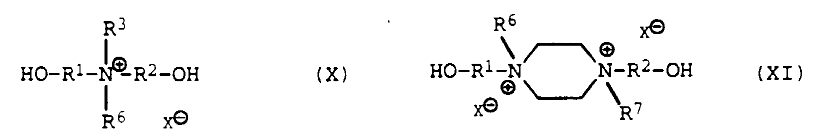 in denen
R¹ und R² C₂- bis C₈-Alkylen bedeuten,
R³, R⁶ und R⁷ für C₁- bis C₄-Alkyl, Phenyl oder C₇- bis C₁₀-Phenylalkyl stehen,
R⁴ und R⁵ Wasserstoff oder C₁- bis C₄-Alkyl bezeichnen und
X^{⊖} für Chlorid, Bromid, Iodid, C₁- bis C₄-Alkylsulfat oder die halbe stöchiometrische Menge Sulfat steht,
eingesetzt werden.

## Claims

1. The use of cationic polyurethanes and polyureas formed from
(a) at least one diisocyanate or reaction product thereof with one or more compounds containing two or more active hydrogen atoms per molecule, and
(b) at least one diol, primary or secondary amino alcohol, primary or secondary diamine or primary or secondary triamine each with one or more tertiary, quaternary or protonated tertiary amine nitrogen atoms
and having a glass transition temperature of at least 25°C and an amine number of from 50 to 200, based on the non-quaternized or -protonated compounds, or other salts of these polyurethanes and polyureas, as ingredients of cosmetic and pharmaceutical preparations.

2. A use as claimed in claim 1 wherefor the compounds of group (a) comprise C₂- to C₈-alkylene diisocyanates, C₅- to C₁₀-cycloalkylene diisocyanates, phenylene diisocyanates or (C₁₋ to C₄-alkyl)phenylene diisocyanates, which each may have already been reacted with one or more compounds selected from the group consisting of diols, amino alcohols, diamines, polyesterols, polyamidediamines and polyetherols each with a number average molecular weight of up to 2000, although up to 3 mol% of the last-mentioned compounds may be replaced by triols or triamines and the diols and aminoalcohols may contain one or more tertiary, quaternary or protonated tertiary amine nitrogen atoms.

3. A use as claimed in claim 1 or 2 wherefor the diisocyanate reaction products comprise at least 5 mol% of a polylactate diol of the general formula I 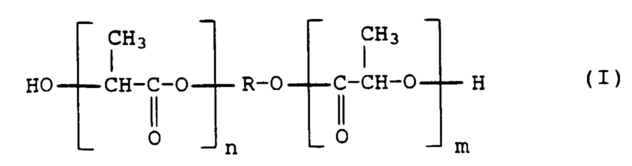 of a poly-ε-caprolactonediol of the general formula II 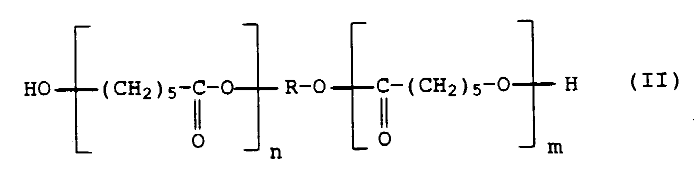 or of a polyamide diamine of the general formula III 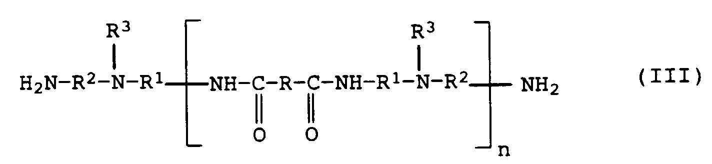 where
R is C₂- to C₈-alkylene, C₅- to C₈-cycloalkylene or phenylene,
R¹ and R² are each C₂- to C₈-alkylene,
R³ is C₁- to C₄-alkyl, phenyl or C₇- to C₁₀-phenylalkyl, and
n and m are each from 1 to 30.

4. A use as claimed in claim 1 wherefor the compounds of group (b) comprise one or more compounds of the general formulae IV to XI 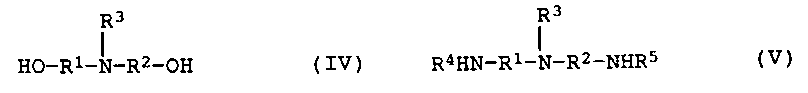 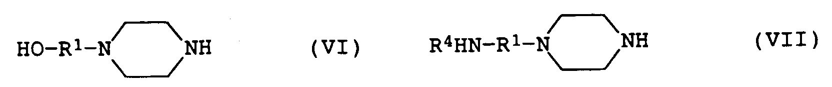 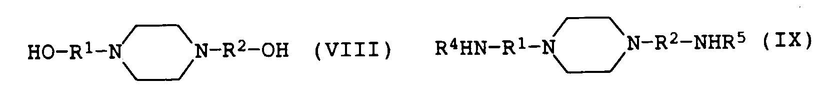 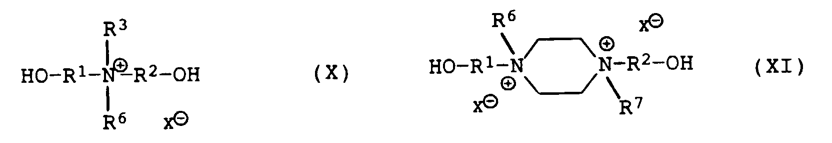 where
R¹ and R² are each C₂- to C₈-alkylene,
R³, R⁶ and R⁷ are each C₁- to C₄-alkyl, phenyl or C₇- to C₁₀-phenylalkyl,
R⁴ and R⁵ are each hydrogen or C₁- to C₄-alkyl, and
X^{⊖} is chloride, bromide, iodide, C₁- to C₄-alkyl sulfate or half the stoichiometric amount of sulfate.

5. Cosmetic and pharmaceutical preparations comprising effective amounts of cationic polyurethanes or polyureas formed from
(a) at least one diisocyanate or reaction product thereof with one or more compounds containing two or more active hydrogen atoms per molecule, and
(b) at least one diol, primary or secondary amino alcohol, primary or secondary diamine or primary or secondary triamine each with one or more tertiary, quaternary or protonated tertiary amine nitrogen atoms
and having a glass transition temperature of at least 25°C and an amine number of from 50 to 200, based on the non-quaternized or -protonated compounds, or other salts of these polyurethanes and polyureas.

6. Cationic polyurethanes and polyureas formed from
(a) C₂- to C₈-alkylene diisocyanates, C₅- to C₁₀-cycloalkylene diisocyanates, phenylene diisocyanates or (C₁- to C₄-alkyl)phenylene diisocyanates, which each may have already been reacted with one or more compounds selected from the group consisting of diols, amino alcohols, diamines, polyesterols, polyamidediamines and polyetherols each with a number average molecular weight of up to 2000, although up to 3 mol% of the last-mentioned compounds may be replaced by triols or triamines and the diols and aminoalcohols may contain one or more tertiary, quaternary or protonated tertiary amine nitrogen atoms and at least 5 mol% of the compounds previously reacted with the diisocyanates comprise a polylactate diol of the general formula I 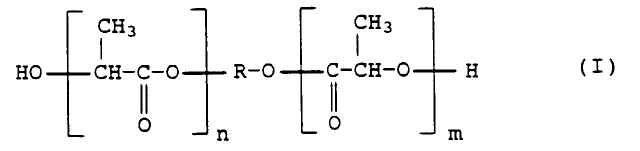 a poly-ε-caprolactonediol of the general formula II 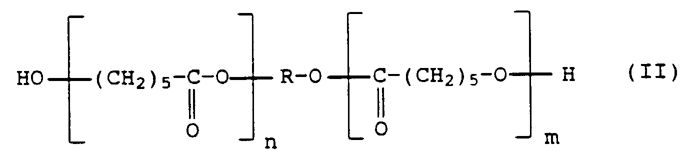 or a polyamide diamine of the general formula III 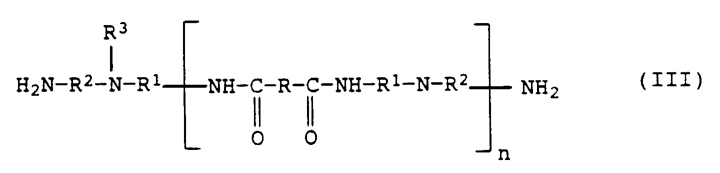 where
R is C₂- to C₈-alkylene, C₅- to C₈-cycloalkylene or phenylene,
R¹ and R² are each C₂- to C₈-alkylene,
R³ is C₁- to C₄-alkyl, phenyl or C₇- to C₁₀-phenylalkyl, and
n and m are each from 1 to 30,
and
(b) at least one diol, primary or secondary amino alcohol, primary or secondary diamine or primary or secondary triamine each with one or more tertiary, quaternary or protonated tertiary amine nitrogen atoms
and having a glass transition temperature of at least 25°C and an amine number of from 50 to 200, based on the non-quaternized or -protonated compounds, or other salts of these polyurethanes and polyureas.

7. Cationic polyurethanes and polyureas as claimed in claim 6 wherein the compounds of group (b) comprise one or more compounds of the general formulae IV to XI 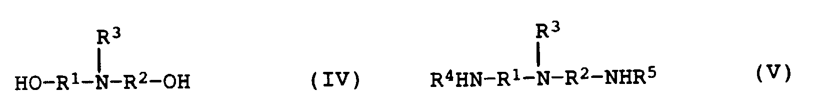 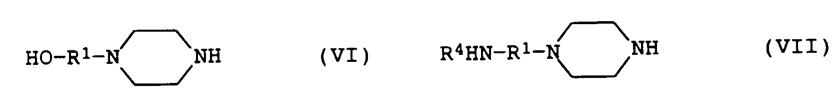 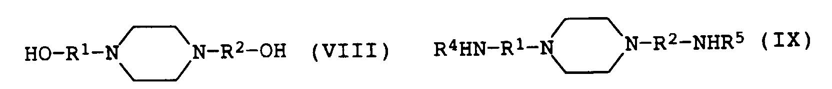 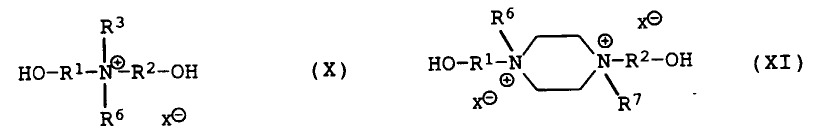 where
R¹ and R² are each C₂- to C₈-alkylene,
R³, R⁶ and R⁷ are each C₁- to C₄-alkyl, pnenyi or C₇- to C₁₀-phenylalkyl,
R⁴ and R⁵ are each hydrogen or C₁- to C₄-alkyl, and
X^{⊖} is chloride, bromide, iodide, C₁- to C₄-alkyl sulfate or half the stoichiometric amount of sulfate.

## Revendications

1. Utilisation de polyurées et de polyuréthannes cationiques, constitués
(a) d'au moins un diisocyanate, qui peut avoir déjà réagi au préalable avec un ou plusieurs composés qui contiennent deux ou plus de deux atomes d'hydrogène actifs par molécule, et
(b) d'au moins une triamine primaire ou secondaire, une diamine primaire ou secondaire, un aminoalcool primaire ou secondaire, un diol, contenant un ou plusieurs atomes d'azote aminés tertiaires, quatemaires ou protonés,
d'une température de transition vitreuse d'au moins 25°C et d'un indice d'amine de 50 à 200, par rapport aux composés non quatemisés ou protonés, ou de certains sels de ces polyuréthannes et polyurées, à titre d'adjuvants dans des préparations cosmétiques et pharmaceutiques.

2. Utilisation suivant la revendication 1, où à titre de composés du groupe (a), on utilise des diisocyanates d'alkylène en C₂ à C₈, des diisocyanates de cycloalkylène en C₅ à C₁₀, des diisocyanates de phénylène, ou des diisocyanates d'(alkyle en C₁ à C₄)phénylène, qui peuvent déjà avoir réagi au préalable avec un ou plusieurs composés appartenant au groupe des diols, aminoalcools, diamines, polyestérols, polyamidodiamines et polyétherols, d'un poids moléculaire moyen en nombre allant à chaque fois jusqu'à 2000, où jusqu'à 3% molaires des composés cités en demier lieu peuvent être remplacés par des triols ou des triamines et les diols et aminoalcools peuvent contenir un ou plusieurs atomes d'azote aminés tertiaires, quatemaires, ou protonés.

3. Utilisation suivant la revendication 1 ou 2, où, à titre des composés ayant déjà réagi au préalable avec des diisocyanates, on utilise au moins 5% molaires d'un poly(esterdiol d'acide lactique) de la formule générale I: 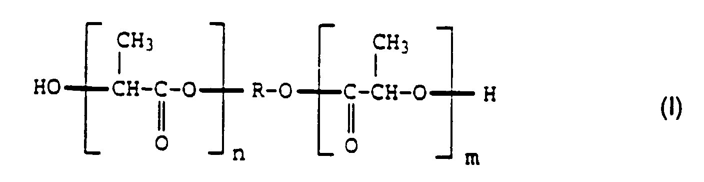 d'un poly(ε-caprolactonediol) de la formule générale II : 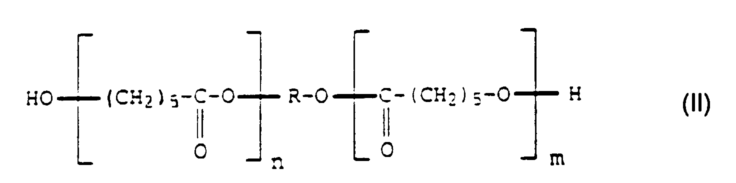 ou d'une polyamidodiamine de la formule générale III : 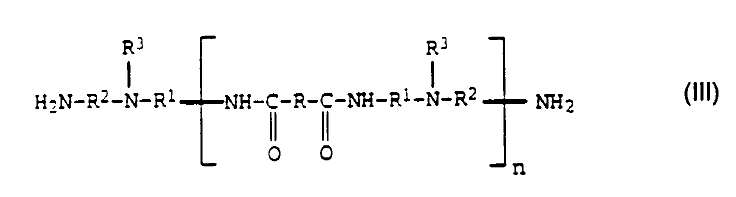 formules dans lesquelles
R représente un radical alkylène en C₂ à C₈, cycloalkylène en C₅ à C₈, ou phénylène,
R¹ et R² représentent des radicaux alkylène en C₂ à C₈,
R³ représente un radical alkyle en C₁ à C₄, phényle ou phénylalkyle en C₇ à C₁₀, et
n et m représentent chacun un nombre qui varie de 1 à 30.

4. Utilisation suivant la revendication 1, où, à titre de composés du groupe (b), on utilise un ou plusieurs composés des formules générales IV à XI : 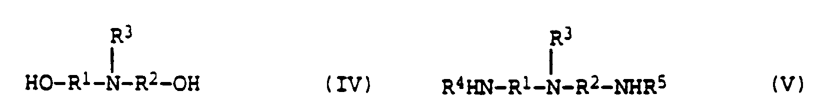 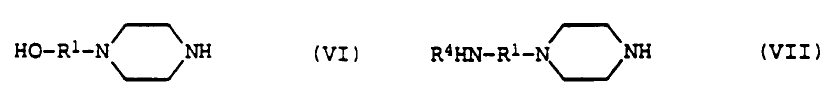 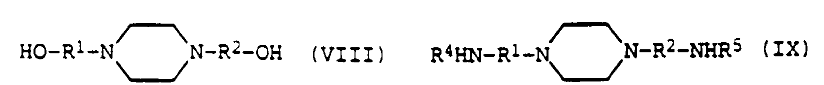 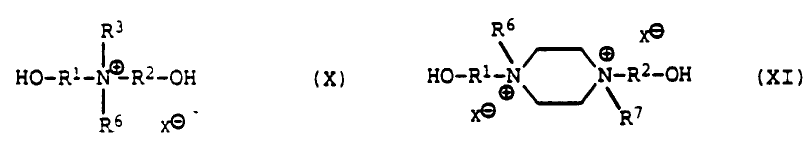 dans lesquelles
R¹ et R² représentent des radicaux alkylène en C₂ à C₈,
R³, R⁶ et R⁷ représentent des radicaux alkyle en C₁ à C₄, phényle ou phénylalkyle en C₇ à C₁₀,
R⁴ et R⁵ représentent des atomes d'hydrogène ou des radicaux alkyle en C₁ à C₄, et
X^{⊖} représente un groupe chlorure, bromure, iodure, alkylsulfate en C₁ à C₄, ou la demi-quantité stoechiométrique de sulfate.

5. Préparations cosmétiques et pharmaceutiques, qui contiennent, à titre d'adjuvant et en proportions actives, des polyurées ou des polyuréthannes cationiques
(a) d'au moins un diisocyanate, qui peut avoir déjà réagi au préalable avec un ou plusieurs composés qui contiennent deux ou plus de deux atomes d'hydrogène actifs par molécule, et
(b) d'au moins une triamine primaire ou secondaire, une diamine primaire ou secondaire, un aminoalcool primaire ou secondaire, un diol, contenant un ou plusieurs atomes d'azote aminés tertiaires, quatemaires ou protonés,
d'une température de transition vitreuse d'au moins 25°C et d'un indice d'amine de 50 à 200, par rapport aux composés non quatemisés ou protonés, ou de certains sels de ces polyuréthannes et polyurées.

6. Polyurées et polyuréthannes cationiques constitués
(a) de diisocyanates d'alkylène en C₂ à C₈, de diisocyanates de cycloalkylène en C₅ à C₁₀, de diisocyanates de phénylène ou de diisocyanates d'(alkyle en C₁ à C₄)phénylène qui peuvent déjà avoir réagi avec un ou plusieurs composés appartenant au groupe des diols, aminoalcools, diamines, polyestérols, polyamidodiamines et polyéthérols, d'un poids moléculaire moyen en nombre allant chaque fois jusqu'à 2000, où jusqu'à 3% molaires des composés cités en demier lieu peuvent être remplacés par des triols ou des triamines, les diols et les aminoalcools peuvent contenir un ou plusieurs atomes d'azote aminés tertiaires, quatemaires ou protonés et où au moins 5% molaires des composés ayant déjà au préalable réagi avec des diisocyanates représentent un poly(esterdiol d'acide lactique) de la formule générale I: 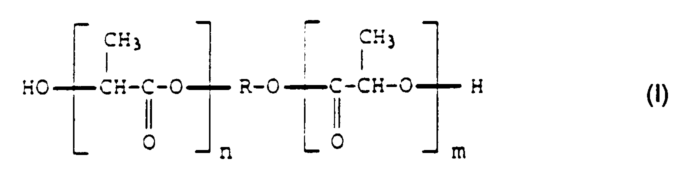 d'un poly(ε-caprolactonediol) de la formule générale II : 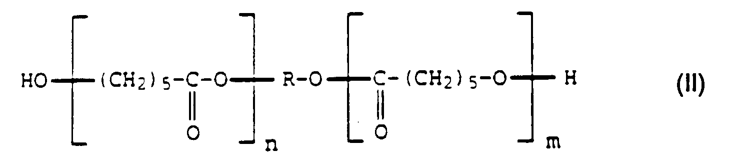 ou d'une polyamidodiamine de la formule générale III : 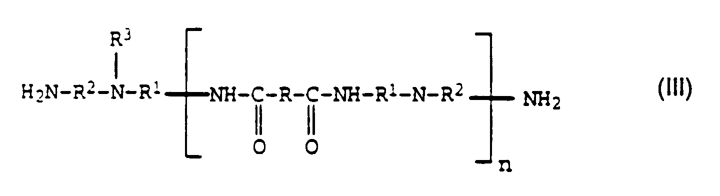 formules dans lesquelles
R représente un radical alkylène en C₂ à C₈, cycloalkylène en C₅ à C₈, ou phénylène,
R¹ et R² représentent des radicaux alkylène en C₂ à C₈,
R³ représente un radical alkyle en C₁ à C₄, phényle ou phénylalkyle en C₇ à C₁₀, et
n et m représentent chacun un nombre qui varie de 1 à 30,
et
(b) d'au moins une triamine primaire ou secondaire, une diamine primaire ou secondaire, un aminoalcool primaire ou secondaire ou d'un diol, contenant un ou plusieurs atomes d'azote aminés tertiaires, quatemaires ou protonés,
d'une température de transition vitreuse d'au moins 25°C et d'un indice d'amine de 50 à 200, par rapport aux composés non quatemisés ou protonés et de certains sels de ces polyuréthannes et polyurées.

7. Polyurées et polyuréthannes cationiques suivant la revendication 6, où, à titre de composés du groupe (b), on utilise un ou plusieurs composés des formules générales IV à XI : 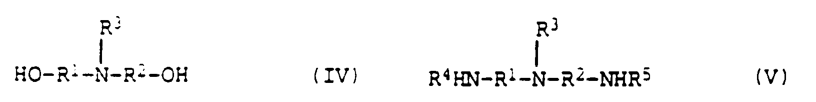 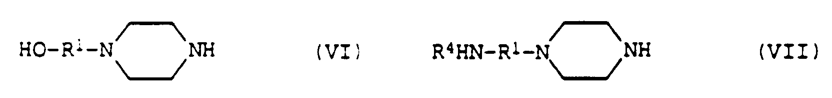 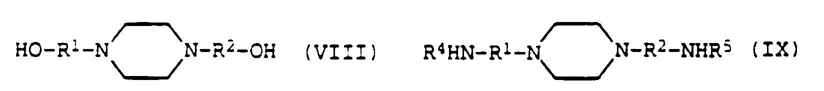 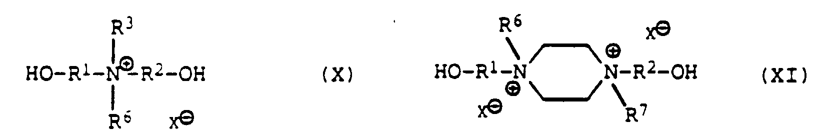 dans lesquelles
R¹ et R² représentent des radicaux alkylène en C₂ à C₈,
R³, R⁶ et R⁷ représentent des radicaux alkyle en C₁ à C₄, phényle ou phénylalkyle en C₇ à C₁₀,
R⁴ et R⁵ représentent des atomes d'hydrogène ou des radicaux alkyle en C₁ à C₄, et
X^{⊖} représente un groupe chlorure, bromure, iodure, alkylsulfate en C₁ à C₄, ou la demi-quantité stoechiométrique de sulfate.
